# EUROPEAN PATENT APPLICATION

(11) **EP 4 578 936 A1**
(43) Date of publication of application: **02.07.2025**
(21) Application number: 24219381.1
(22) Date of filing: 12.12.2024
(51) Int. Cl.: C12M 1/00, C12M 1/12, C12M 1/26, C12N 1/02, C12Q 1/24, C12Q 1/68

(54) **APPARATUS AND METHOD FOR MICROORGANISM DETECTION INVOLVING FILTRATION**

(30) Priority: 27.12.2023 JP 2023221156
(71) Applicant: Yokogawa Electric Corporation, Tokyo 180-8750 (JP)
(72) Inventor: HIRAKAWA, Yuko, Musashino-shi, Tokyo, 180-8750 (JP); MITSUMORI, Yuuji, Musashino-shi, Tokyo, 180-8750 (JP); UCHINO, Youichi, Musashino-shi, Tokyo, 180-8750 (JP); TAGUCHI, Tomoyuki, Musashino-shi, Tokyo, 180-8750 (JP)
(74) Representative: Hoffmann Eitle

(57) **Abstract**

Provided is an apparatus including: a first filtration unit that filters a fluid sample by a filter; a culture unit that performs culture using a resultant object that has undergone a processing treatment to reduce a flat size for the filter after filtration by the first filtration unit; a detection unit that detects nucleic acids of organisms cultured by the culture unit; and a processing treatment unit that performs the processing treatment on the filter after filtration by the first filtration unit, and produces a filter processed object as the resultant object, wherein the processing treatment unit cuts the filter after filtration by the first filtration unit to produce the filter processed object.

## Description

### BACKGROUND

### 1. TECHNICAL FIELD

The present invention relates to an apparatus and a method.

### 2. RELATED ART

In Patent Documents 1 to 5, it is described that "the microorganisms are collected on a membrane filter with an outer frame, and the microorganisms are cultured, immobilized, hybridized with a fluorescent probe, washed, and observed with a fluorescence microscope while the microorganisms are maintained on the membrane filter with the outer frame".

### Prior Art Documents

### Patent Documents

Patent Document 1: Japanese Patent Application Publication No. 2006-296285
Patent Document 2: Japanese Patent Application Publication No. 2007-202553
Patent Document 3: Japanese Patent Application Publication No. 2009-034093
Patent Document 4: Japanese Patent Application Publication No. 2010-213724
Patent Document 5: Japanese Patent Application Publication No. 2009-082153
Patent Document 6: International Publication No. 2019/43779
Patent Document 7: Japanese Patent No. 5624487
Patent Document 8: Japanese Patent No. 4857373

### SUMMARY

A first aspect of the present invention provides an apparatus including: a first filtration unit that filters a fluid sample by a filter; a culture unit that performs culture using a resultant object that has undergone a processing treatment to reduce a flat size for the filter after filtration by the first filtration unit; and a detection unit that detects nucleic acids of organisms cultured by the culture unit.

The above-described apparatus further may include a processing treatment unit that performs the processing treatment on the filter after filtration by the first filtration unit, and produces a filter processed object as the resultant object.

In the above-described apparatus including the processing treatment unit, the processing treatment unit may cut the filter after filtration by the first filtration unit to produce the filter processed object.

In the above-described apparatus including the processing treatment unit, the processing treatment unit may bend the filter after filtration by the first filtration unit to produce the filter processed object. Bending the filter may mean bending the filter so that the front and back surfaces are in face contact, in other words, bending the filter to 180 degrees, or bending the filter at an angle of less than 180 degrees. The bending line of the filter may be preset based on the vertical direction or the horizontal direction of the filter, or they may be random. In the culture unit, The flat shape of the culture tank may be smaller than the flat shape of the filter, or may be smaller than the cross section of the flow channel in the first filtration unit. As long as the filter processed object is contained in the culture tank along the bottom of the culture tank, the flat shape of the filter processed object may be the same as the flat shape of the culture tank, or it may be a similar shape smaller than the flat shape of the culture tank.

The above-described apparatus including the processing treatment unit may further include a second filtration unit that filters a liquid medium after culture using the filter processed object; and an extraction unit that extracts nucleic acids from a residue after filtration by the second filtration unit. The detection unit may detect nucleic acids extracted by the extraction unit.

In the above-described apparatus, the extraction unit may contain the filter processed object used in filtration by the second filtration unit in a sealed container for heating, and extract nucleic acids of organisms.

Any of the above-described apparatus may further include a third filtration unit that filters a fluid sample by a pre-filter with meshes larger than that of the filter before filtration by the first filtration unit.

A second aspect in the present invention provides a method including: first filtering for filtering a fluid sample by a filter; culturing using a resultant object that has undergone a processing treatment to reduce a flat size for the filter after filtration; and detecting nucleic acids of organisms cultured by the culturing.

Note that the summary clause does not necessarily describe all necessary features of the embodiments of the present invention. The present invention may also be a sub-combination of the features described above.

### BRIEF DESCRIPTION OF THE DRAWINGS

Fig. 1 illustrates a microorganism detection apparatus 1 according to an embodiment.
Fig. 2 illustrates states of a filter 101 and a filter processed object 102 in a first filtration unit 12, a processing treatment unit 13, a culture unit 14, a second filtration unit 15 and a nucleic acid extraction unit 16.
Fig. 3 illustrates operations of the microorganism detection apparatus 1.

### DESCRIPTION OF EXEMPLARY EMBODIMENTS

Hereinafter, embodiments of the present invention will be described. However, the following embodiments are not for limiting the invention according to the claims. In addition, not all of the combinations of features described in the embodiments are essential to the solution of the invention.

### < 1. Microorganism Detection Apparatus>

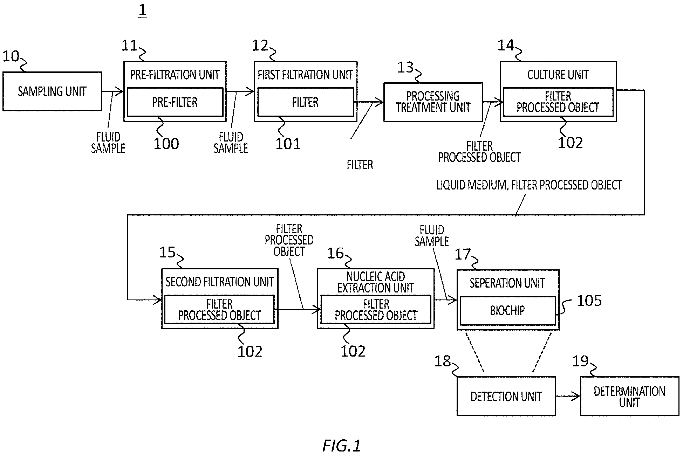

Fig. 1 illustrates a microorganism detection apparatus 1 according to the present embodiment. The microorganism detection apparatus 1 may be an example of an apparatus, which detects microorganisms mixed in food or beverages. The microorganism detection apparatus 1 includes a sampling unit 10, a pre-filtration unit 11, a first filtration unit 12, a processing treatment unit 13, a culture unit 14, a second filtration unit 15, a nucleic acid extraction unit 16, a separation unit 17, a detection unit 18 and a determination unit 19.

The microorganisms to be detected can be selected from a group consisting of, as an example, *Acinetobacter* species, *Actinomyces* species, *Aerococcus* species, *Aeromonas* species, *Alcaligenes* species, *Bacillus* species, *Bacteriodes* species, *Bordetella* species, *Branhamella* species, *Brevibacterium* species, *Campylobacter* species, *Candida* species, *Capnocytophaga* species, *Chromobacterium* species, *Clostridium* species, *Corynebacterium* species, *Cryptococcus* species, *Deinococcus* species, *Enterococcus* species, *Erysipelothrix* species, *Escherichia* species, *Flavobacterium* species, *Gemellα* species, *Haemophilus* species, *Klebsiella* species, *Lactobacillus* species, *Lactococcus* species, *Legionella* species, *Leuconostoc* species, *Listeria* species, *Micrococcus* species, *Mycobacterium* species, *Neisseria* species, *Cryptosporidium* species, *Nocardia* species, *Oerskovia* species, *Paracoccus* species, *Pediococcus* species, *Peptostreptococcus* species, *Propionibacterium* species, *Proteus* species, *Pseudomonas* species, *Rahnella* species, *Rhodococcus* species, *Rhodospirillium* species, *Staphylococcus* species, *Streptomyces* species, *Streptococcus* species, *Vibrio* species and *Yersinia* species. There are microorganisms that take the form of blasts and spores in oligotrophic conditions, regardless of the cellular status due to such growth conditions.

### <1.1. Sampling Unit 10>

The sampling unit 10 extracts a sample from food or a beverage. The sampling unit 10 may extract a sample by suctioning the beverage or may extract a sample by suctioning and filtering the beverage through a filter (also referred to as a membrane filter). The sampling unit 10 may crush the food to extract a sample. The sampling unit 10 may supply the sample as a fluid sample to the pre-filtration unit 11. When extracting the sample obtained by crushing the food, the sampling unit 10 may supply the sample as a fluid sample in saline or liquid medium to the pre-filtration unit 11.

### <1.2. Pre-filtrationUnit 11>

The pre-filtration unit 11 is an example of a third filtration unit, which filters the fluid sample with a pre-filter 100 before filtration by the first filtration unit 12. The pre-filter 100 may have larger meshes (also referred to as filter pore size) than those of the filter 101 described below, which is used in the first filtration unit 12. For example, the pre-filtration unit 11 may coarsely filter the fluid sample, and the pre-filter 100 may have larger meshes than 5 µm. The pre-filter 100 may remove foreign substances other than the microorganisms from the fluid sample. The material of the pre-filter 100 is not limited particularly as long as the material does not interfere with filtration in the first filtration unit 12 or culture in the culture unit 14. For the material of the pre-filter 100, cellulose fiber (as an example, ordinary filter paper), polyester or PET (as an example, mesh made of polyester or PET) may be used, in addition to any of the materials described as materials for the filter 101 described below. The pre-filtration unit 11 may supply the fluid sample after filtration to the first filtration unit 12.

### <1.3. First Filtration Unit 12>

The first filtration unit 12 filters the fluid sample by the filter 101.

The filter 101 used for filtration preferably has a pore size capable of capturing microorganisms to be detected. For example, the first filtration unit 12 may microfilter the fluid sample, and the filter 101 preferably has meshes of 0.45 µm or less. The filter 101 may be a screen filter or a depth filter, but in the present embodiment it is a screen filter as an example. The flat shape of the filter 101 may be the same as the cross-sectional shape of the flow channel in the first filtration unit 12, as long as the filter 101 is not flushed by the fluid sample in the first filtration unit 12, and may be circular as an example.

The material of the filter 101 is not particularly limited as long as the material does not interfere with the extraction of the nucleic acids in the nucleic acid extraction unit 16 described below and the extracted nucleic acids are not easily adsorbed. For example, the material of the filter 101 may be polytetrafluoroethylene (PTFE), polyvinylidene fluoride (PVDF), polyethersulfone (PES), cellulose mixed ester, polycarbonate (PC), nylon, polyvinyl chloride (PVC), pure silver, etc. The term "cellulose mixed ester" is a biologically inert material consisting of a mixture of cellulose acetate and cellulose nitrate. The material of the filter 101 is preferably hydrophilic, for example, hydrophilic PTFE, hydrophilic PVDF, hydrophilic PES or hydrophilic PC preferably. When the filter 101 is hydrophilic, nucleic acids extracted in the nucleic acid extraction unit 16 described below are not easily adsorbed on the filter 101. Among the materials, hydrophilic PC is more preferred as the material of the filter 101. When the material of the filter 101 is hydrophilic PC, the pore size or the pore size distribution tends to be constant. By using the filter 101 with constant pore size or pore size distribution, microorganisms can be collected stably. One indicator indicative of the hydrophilic nature of the material of the filter 101 can be the contact angle as an example. For example, a filter made by Merck Corporation (Millipore Corporation) or a filter made by Advantech Toyo Corporation can be used as the above filter. The first filtration unit 12 may supply the filter 101 with microorganisms captured as a residue to the processing treatment unit 13.

### <1.4. Processing Treatment Unit 13>

The processing treatment unit 13 performs a processing treatment to reduce a flat size for the filter 101 after filtration by the first filtration unit 12, and produces the filter processed object 102. The processing treatment unit 13 may process the filter 101 supplied from the first filtration unit 12 to produce the filter processed object 102. The processing treatment unit 13 may cut the filter 101 after filtration by the first filtration unit 12 to produce the filter processed object 102. Additionally or alternatively, the processing treatment unit 13 may bend the filter 101 after filtration by the first filtration unit 12 to produce the filter processed object 102.

The processing treatment unit 13 may produce the filter processed object 102 to a size that allows the filter processed object 102 to be placed on the solid medium in the culture unit 14 or a size that allows the filter processed object 102 to be immersed in the liquid medium in the culture unit 14, for example, produce the filter processed object 102 to fit the dimensions of the culture tank (not illustrated). As an example, the processing treatment unit 13 may cut (i.e., cut out) or may bend the filter 101 using a mold preformed to fit the flat shape of the culture tank (for example, bottom shape). Bending the filter 101 may mean bending the filter 101 so that the front and back surfaces are in face contact, in other words, bending the filter 101 to 180 degrees, or bending the filter 101 at an angle of less than 180 degrees. The bending line of the filter 101 may be preset based on the vertical direction or the horizontal direction of the filter 101, or may be random. As an example, the filter processed object 102 may be produced by crumpling the filter 101 into a crumpled round. The flat shape of the culture tank may be smaller than the flat shape of the filter 101, or may be smaller than the cross section of the flow channel in the first filtration unit 12. As long as the filter processed object 102 is contained in the culture tank along the bottom of the culture tank, the flat shape of the filter processed object 102 may be the same as the flat shape of the culture tank, or may be a similar shape smaller than the flat shape of the culture tank.

Additionally, the processing treatment unit 13 may produce the filter processed object 102 so that it can be used as a filter in the second filtration unit 15, or may produce the filter processed object 102 to fit the cross-sectional shape of the flow channel of the second filtration unit 15. In this case, the processing treatment unit 13 may cut (i.e., cut out) or may bend the filter 101 using a mold preformed to fit the cross-sectional shape of the flow channel of the second filtration unit 15. The cross-sectional shape of the flow channel in the second filtration unit 15 may be smaller than the cross-sectional shape of the flow channel in the first filtration unit 12 and the flat shape of the culture tank. The flat shape of the filter processed object 102 may be the same as the cross-sectional shape of the flow channel of the second filtration unit 15, for example, it may be circular, as long as the filter processed object 102 is not flushed by the fluid sample in the second filtration unit 15.

The processing treatment unit 13 may process the filter 101 in a wet state due to the liquid medium to produce the filter processed object 102, or may dry the filter 101 and then cut it to produce the filter processed object 102. The processing treatment unit 13 may discard the remaining portion of the filter 101 that was not used as the filter processed object 102. The processing treatment unit 13 may supply the produced filter processed object 102 to the culture unit 14.

### <1.5. Culture Unit 14>

The culture unit 14 performs culture using the resultant object that has undergone a processing treatment to reduce a flat size for the filter 101, which is produced from the filter 101 after filtration. The culture unit 14 may perform culture using the filter processed object 102 produced by the processing treatment unit 13.

The culture unit 14 may culture the microorganisms captured by the filter 101. The culture unit 14 may perform culture under any conventionally well known method or conditions according to the microorganisms to be detected. For example, the culture unit 14 may culture the sample on a solid medium (i.e., solid phase culture), and as an example, the filter processed object 102 may be cultured on a solid medium. Alternatively, the culture unit 14 may culture the sample inside a liquid medium (i.e., liquid phase culture), and as an example, the filter processed object 102 may be cultured by being immersed in the liquid medium. The liquid medium may be a solution of dissolved solid medium.

Herein, because the filter processed object 102 has a smaller flat size than that of the filter 101, the culture tank in the present embodiment that contains the filter processed object 102 may have a smaller flat shape compared to the culture tank that contains the filter 101 (also referred to as a conventional culture tank). This allows the medium in the culture tank according to the present embodiment to have a smaller flat dimension compared to the medium in a conventional culture tank. However, a thickness of the solid medium (or a depth of the liquid medium) in the culture tank according to the present embodiment may be the same as a thickness of the solid medium (or a depth of the liquid medium) in a conventional culture tank.

Therefore, in the culture tank according to the present embodiment, the volume of the medium may be smaller compared to the conventional culture tank. On the other hand, in the culture tank according to the present embodiment, microorganisms may be culturable in the same amount as the conventional culture tank. This allows microorganisms to be cultured in the culture tank according to the present embodiment at a higher concentration compared to the conventional culture tank.

The culture unit 14 may supply the liquid medium after culture to the second filtration unit 15. The culture unit 14 may dissolve the solid medium and supply it to the second filtration unit 15 as a liquid medium when performing a solid phase culture.

### <1.6. Second Filtration Unit 15>

The second filtration unit 15 filters the liquid medium after culture using the filter processed object 102. In this manner, the cultured microorganisms are collected as a residue.

The second filtration unit 15 may supply the collected microorganisms to the nucleic acid extraction unit 16. In the present embodiment, as an example, the second filtration unit 15 may supply the microorganisms collected as the residue on the filter processed object 102 to the nucleic acid extraction unit 16 with the entire filter processed object 102.

### <1.7. Nucleic Acid Extraction Unit 16>

The nucleic acid extraction unit 16 extracts nucleic acids (genomic DNA, ribosomal RNA, plasmid DNA or the like as an example) from the residue after filtration by the second filtration unit 15, as an example of the extraction unit. The nucleic acid extraction unit 16 may contain the filter used in the filtration by the second filtration unit (the filter processed object 102 as an example in the present embodiment) in a container that is sealed (also referred to as a sealed container) for heating and extract nucleic acids of the microorganisms, or may use, for example, the method according to International Publication No. 2019/43779 or Japanese Patent No. 5624487, which is a so-called dHTP method to extract the nucleic acids.

The nucleic acid extraction unit 16 may expose microorganisms as a residue to high temperature conditions with the entire filter processed object 102 in the sealed container to destroy the membrane structures of the microorganisms and make the nucleic acids in an extractable state. The container may be, for example, a thermally fusible bag or a boil lock tube. The container may be, for example, a plastic tube, a glass test tube, or a microfluidic chip.

The container may be heated by heating means not shown. The heating means may be an oil bath or a heat block, which may heat the inside of the container to a temperature of up to 200°C. The pressure in the container may become atmospheric pressure or higher due to heating.

At least one cell dissolution promoter selected from the group consisting of an alkali, acid, enzyme, surfactant, redox agent or protein denaturing agent having the ability to dissolve membrane structures may be added into the container.

For example, sodium hydroxide (NaOH), potassium hydroxide (KOH) or the like can be used as the above-described alkali. For example, hydrochloric acid (HCl), sulfuric acid (H₂SO₄) or the like can be used as the above-described acid. For example, a proteinase enzyme such as Proteinase K, or a polysaccharide-degrading enzyme such as chitinase, lysozyme, zymolyase, etc. can be used as the above-described enzyme. The above-described surfactant may be ionic or nonionic, for example. For example, octylphenol ethoxylate (C₁₄H₂₂O(C₂H₄O)ₙ) or the like can be used as the nonionic surfactant. For example, commercial products such as TritonX-100 (C₁₄H₂₂O(C₂H₄O)ₙ, n=100) made by SIGMA Corporation can be used as the octylphenol ethoxylate.

The ionic surfactant may be anionic, may be cationic, or may be amphionic. For example, sodium dodecyl sulfate (SDS) or the like can be used as an anionic surfactant. For example, cetyltrimethylammonium bromide (CATB) or the like can be used as a cationic surfactant. For example, betaine or the like can be used as an amphionic surfactant. Herein, "betaine" is a generic term for compounds that have a positive charge and a negative charge at non-adjacent positions in the same molecule, have no dissociable hydrogen atom bonded to the atom with a positive charge, and have no charge in the molecule as a whole. Trimethylglycine can be used as a typical example of betaine. For example, hydrogen peroxide, β-mercaptoethanol, dithiothreitol or the like can be used as the above-described redox agent.

For example, guanidine hydrochloride, urea or the like can be used as the above-described protein denaturing agent. For example, ethylenediaminetetraacetic acid (EDTA) or the like can be used as the above-described chelating agent. Among the above-mentioned cell dissolution promoters, it is preferred that the above-described surfactant is added, and it is more preferred that either one or both of SDS and octylphenol ethoxylate are added. For example, when nucleic acids to be extracted is to be detected with high sensitivity, SDS may be used. In contrast, when the extracted nucleic acids are to be used for enzymatic reactions that are inhibited by SDS, octylphenol ethoxylate, which acts more mildly than SDS on membrane structures of microorganisms, may be used.

The container may further contain a buffer solution inside. For example, tris (hydroxymethyl) aminomethane hydrochloride (Tris-HCl) or the like can be used as the buffer solution.

The nucleic acid extraction unit 16 may extract nucleic acids from the liquid inside the container after the membrane structures of microorganisms have been destroyed. The nucleic acid extraction unit 16 may bond nucleic acids with magnetic microparticles to extract the nucleic acids by magnetism by, for example, the method according to Japanese Patent No. 4857373. Alternatively, the nucleic acid extraction unit 16 may extract nucleic acids by at least one of filtration, centrifugation or electrophoresis. When nucleic acids are extracted by electrophoresis, the nucleic acids may be charged beforehand by well known methods.

The nucleic acid extraction unit 16 may supply the fluid sample containing the extracted nucleic acids to the separation unit 17. The nucleic acid extraction unit 16 may supply the fluid sample with amplified nucleic acids to the separation unit 17. The nucleic acid extraction unit 16 may amplify nucleic acids in the liquid in a state where the membrane structures of microorganisms has been destroyed, or may extract and then amplify the nucleic acids after extracting the nucleic acids. The nucleic acid extraction unit 16 may amplify nucleic acids by the method of polymerase chain reaction (PCR).

### <1.8. Separation Unit 17>

The separation unit 17 separates nucleic acids of the microorganisms to be detected from the nucleic acids in the fluid sample. The separation unit 17 may separate nucleic acids that are unique to the microorganisms to be detected (also referred to as target nucleic acids) from the nucleic acids of the microorganisms to be detected. The separation unit 17 may separate the target nucleic acids using the biochip 105.

The biochip 105 may have a plurality of probes that each hybridizes with a corresponding base sequence. At least some of the plurality of probes may have a base sequence complementary to at least a portion of the base sequence of the target nucleic acid or may specifically hybridize with the nucleic acid of the corresponding base sequence. At least some of the plurality of probes may have the same base sequence.

Each probe may be pre-fixed at a unique position within the biochip 105. As an example in the present embodiment, the biochip 105 may have a plurality of probes on the inner surfaces of two transparent substrates arranged opposite each other. The biochip 105 may have an inlet for implanting a fluid sample and an outlet for draining the fluid sample inside.

At least one of the probes of the biochip 105 having a complementary base sequence or the nucleic acids in the fluid sample may be pre-attached with a label that emits a signal detectable by the detection unit 18 described below in a hybridized state. For example, the label may be attached to the probe and may emit a signal according to the hybridization between the probe and the nucleic acids. Alternatively, the label may be attached to nucleic acids in the fluid sample and may emit the signal regardless of whether the nucleic acids hybridize with the probe. In this case, after the implantation of the fluid sample into the biochip 105, nucleic acids that have not hybridized with the probe may be removed from the biochip 105, and then the signal may be detected. A fluorescent dye, a radioisotope or a paramagnetic isotope, an enzyme or the like can be used as the label. As an example in the present embodiment, the label may be a fluorescent dye, which may be attached to the probe.

### <1.9. Detection Unit 18>

The detection unit 18 detects nucleic acids of the microorganisms cultured by the culture unit 14. The detection unit 18 may detect nucleic acids extracted by the nucleic acid extraction unit 16. The detection unit 18 may detect nucleic acids by detecting the labels inside the biochip 105. The detection unit 18 may be disposed opposite the biochip 105 and may detect the label inside the biochip 105 via the transparent substrate of the biochip 105. The detection unit 18 may detect a signal emitted from a label attached to either one of the hybridized probes or the nucleic acids. The detection unit 18 may supply information indicating the position of the detected label, or the intensity of the signal emitted from the label (fluorescence intensity as an example in the present embodiment) to the determination unit 19.

### <1.10. Determination Unit 19>

The determination unit 19 determines whether the fluid sample implanted into the biochip 105 contained more nucleic acids of microorganisms to be detected than the reference value, or in other words, whether the fluid sample was any of positive or negative. The determination unit 19 may perform determination based on the information supplied from the detection unit 18. For example, the determination unit 19 may perform determination according to a detected position of the label by the detection unit 18. The determination unit 19 may output the determination result indicating whether the fluid sample was any of positive or negative to the outside.

The reference value may indicate the number of nucleic acids, or may be a number of labels as target nucleic acids detected by the detection unit 18. As an example in the present embodiment, the reference value may be 0. However, the reference value may indicate a proportion of nucleic acids, or may be a proportion of the labels detected by the detection unit 18 as the target nucleic acids among the number of labels detected by the detection unit 18.

According to the microorganism detection apparatus 1 described above, the area of the medium can be reduced by using the resultant object that has undergone a processing treatment to reduce the flat size for the filter 101 after filtration by the first filtration unit 12 for culture. This allows the microorganisms to be cultured at a high concentration in a narrow medium, thereby increasing the detection accuracy of nucleic acids.

The filter 101 after filtration undergoes a processing treatment by the processing treatment unit 13 to produce the filter processed object 102. Therefore, the process from filtration by the first filtration unit 12 to the culture by the culture unit 14 can be made more efficient compared to the case where the filter processed object 102 is produced from the filter 101 outside the microorganism detection apparatus 1.

Because the filter processed object 102 is produced by cutting the filter 101 after filtration by the first filtration unit 12, the filter processed object 102 with a smaller flat size than that of the filter 101 can be easily produced. Additionally or alternatively, the filter processed object 102 is produced by bending the filter 101 after filtration, the filter processed object 102 with a smaller flat size than the filter 101 can be easily produced.

Because the liquid medium after culture is filtered using the filter processed object 102 produced from the filter 101 that has filtered the liquid sample, the usage amount of the filter can be reduced compared to the case of using a separate filter for the filtration of the liquid sample and the filtration of the liquid medium.

The nucleic acids of microorganisms are extracted by containing the filter processed object 102 that has filtered the liquid medium after culture in a sealed container for heating, thus capable of eliminating the need to separate the microorganisms from the filter processed object 102 and making it easier to obtain the nucleic acids of the microorganisms in the liquid sample.

Because the fluid sample is filtered by the pre-filter 100, which has meshes larger than that of the filter 101 before filtration by the first filtration unit 12, foreign substances in the fluid sample can be removed for performing culture.

### <2. Filter 101 and Filter Processed Object 102>

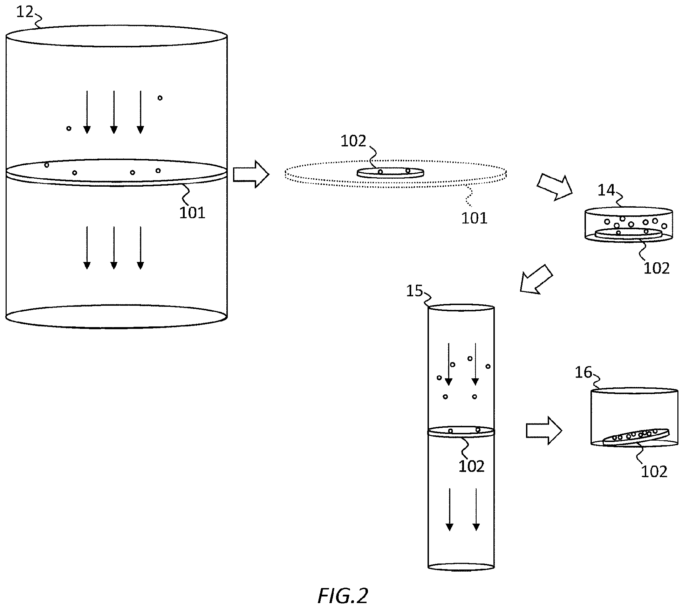

Fig. 2 illustrates the state of the filter 101 and the filter processed object 102 in the first filtration unit 12, the processing treatment unit 13, the culture unit 14, the second filtration unit 15 and the nucleic acid extraction unit 16. The white circle symbols in the figure indicate microorganisms to be detected. The white arrow symbols in the figure indicate the process flow in the microorganism detection apparatus 1.

The filter 101 is used to filter the fluid sample in the first filtration unit 12 to capture microorganisms (see upper left in the figure), and then undergo the processing treatment by the processing treatment unit 13 to become the filter processed object 102 (see upper right in the figure). Then the filter processed object 102 is used for culture in the culture unit 14 (see upper center in the figure) and is used for filtration of the liquid medium in the second filtration unit 15 to capture the microorganisms (see lower center in the figure). The filter processed object 102 is then contained in a sealed container for heating in the nucleic acid extraction unit 16 (see lower right in the figure) to extract the nucleic acids of the microorganisms contained in the sample.

### <3. Operations of Microorganism Detection Apparatus 1>

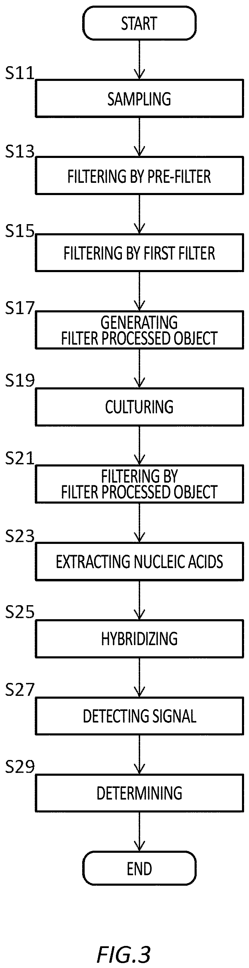

Fig. 3 illustrates operations of the microorganism detection apparatus 1. By performing the process of steps S11 to S29, the microorganism detection apparatus 1 determines whether each sample contains more microorganisms to be detected than the reference value.

In step S11, the sampling unit 10 extracts samples from food and beverages. The sampling unit 10 may produce a fluid sample from the sample.

In step S13, the pre-filtration unit 11 filters the fluid sample by the pre-filter 100. This may remove foreign substances from the fluid sample.

In step S15, the first filtration unit 12 filters the fluid sample by the filter 101. This may capture the microorganisms to be detected as a residue.

In step S17, the processing treatment unit 13 performs a processing treatment to reduce the flat size on the filter 101 after filtration to produce the filter processed object 102. The filter processed object 102 may have captured microorganisms attached on the filter 101 in step S15. The processing treatment unit 13 may be washed after the filter processed object 102 is produced. This prevents microorganisms attached to the filter 101 from remaining in the processing treatment unit 13.

In step S19, the culture unit 14 performs culture using the resultant object that has undergone the processing treatment to reduce a flat size for the filter 101, which is produced from the filter 101 after filtration. As an example in the present embodiment, the culture unit 14 may perform culture using the filter processed object 102. Microorganisms may be captured in the filter processed object 102. In this manner, the microorganisms captured by the filter processed object 102 are then cultured.

In step S21, the second filtration unit 15 filters the liquid medium after culture using the filter processed object 102. This may capture the microorganisms cultured in step S19. The liquid medium filtered by the filter processed object 102 in step S21 may be the liquid medium in which the filter processed object 102 has been immersed in step S19.

In step S23, the nucleic acid extraction unit 16 extracts the nucleic acids of the microorganisms contained in the liquid medium from the residue after filtration by the second filtration unit 15. As an example, the nucleic acid extraction unit 16 may contain the microorganisms as a residue in a container with the entire filter processed object 102, destroy the membrane structures of microorganisms using the so-called dHTP method, and then extract the nucleic acids by filtration or centrifugation or the like.

In step S25, the separation unit 17 separates nucleic acids of the microorganisms to be detected from the nucleic acids in the fluid sample. As an example, the separation unit 17 may separate the nucleic acids of the microorganisms to be detected contained in the fluid sample by hybridizing them with the probe of the biochip 105.

In step S27, the detection unit 18 detects nucleic acids in the cultured microorganisms. The detection unit 18 may detect nucleic acids by detecting the labels in the biochip 105. As an example, the detection unit 18 may detect a signal emitted from a label attached to at least one probe of the biochip 105 and the nucleic acid in the fluid sample.

In step S29, the determination unit 19 determines whether the fluid sample implanted into the biochip 105 contained more nucleic acids of the microorganisms to be detected than the reference value, or in other words, whether the fluid sample was any of positive or negative, based on information supplied from the detection unit 18.

### <4. Modification Example>

In the above-described embodiment, although the microorganism detection apparatus 1 has been described to include the sampling unit 10, the pre-filtration unit 11, the processing treatment unit 13, the second filtration unit 15, the nucleic acid extraction unit 16, the separation unit 17 and the determination unit 19, it may not include either of these. For example, when the microorganism detection apparatus 1 does not include the processing treatment unit 13, the filter processed object 102 may be produced from the filter 101 after filtration by an apparatus outside the microorganism detection apparatus 1 or an operator. When the microorganism detection apparatus 1 does not include the second filtration unit 15 or the nucleic acid extraction unit 16, the nucleic acids of the microorganisms cultured on the solid medium by the culture unit 14 may be detected by the detection unit 18. In this case, as an example, the separation unit 17 may bring a permeability-imparting composition containing polyethyleneimine and at least one alcohol into contact with the filter processed object 102 and microorganisms on the solid medium, and may allow molecules that contain labels and hybridize with target nucleic acids to permeate the interior of the membrane structures of microorganisms after the microorganisms being secured to the filter processed object 102 by a cross-linking agent. This allows the nucleic acids of the cultured microorganisms to be detected. As a method of detecting nucleic acids in this manner, for example, the methods according to the above-mentioned Patent Documents 2 to 4 can be used.

Although the second filtration unit 15 has been described as filtering the liquid medium using the filter processed object 102 produced from the filter 101, other filters different from the filter processed object 102 may be used to filter the liquid medium. The other filter may be a filter similar to the filter 101.

Although the processing treatment unit 13 has been described as performing a processing treatment of at least one of cutting or bending the filter 101 to reduce the flat size, other processes may be used to reduce the flat size. As an example, the processing treatment unit 13 may dissolve at least a portion of the filter 101 to reduce the flat size while keeping the microorganisms captured in the filter 101 in a culturable state. In this case, the culture unit 14 may perform culture using the dissolved solution in which the filter 101 is dissolved.

The determination unit 19 is described as determining whether the sample contains nucleic acids of the microorganisms to be detected, but it may also determine whether the sample contains nucleic acids of organisms other than the microorganisms. In this case, the culture unit 14 may culture cells of the organisms to be detected, and the second filtration unit 15 may collect the cultured cells. The organisms to be detected may be animals, insects, plants, mycoplasmas, viruses or the like. The organisms to be detected may be of one species, or may be of a plurality of species.

While the present invention has been described with the embodiments, the technical scope of the present invention is not limited to the above-described embodiments. It is apparent to persons skilled in the art that various alterations and improvements can be added to the above-described embodiments. It is also apparent from description of the claims that the embodiments to which such modifications or improvements are made may be included in the technical scope of the present invention.

It should be noted that each process of the operations, procedures, steps, stages, and the like performed by the apparatus, system, program, and method shown in the claims, specification, or drawings can be executed in any order as long as the order is not indicated by "prior to", "before", or the like and as long as the output from a previous process is not used in a later process. Even if the operation flow is described using phrases such as "first" or "next" for the sake of convenience in the claims, specification, or drawings, it does not necessarily mean that the process must be performed in this order.

### EXPLANATION OF REFERENCES

1: microorganism detection apparatus; 10: sampling unit; 11: pre-filtration unit; 12: first filtration unit; 13: processing treatment unit; 14: culture unit; 15: second filtration unit; 16: nucleic acid extraction unit; 17: separation unit; 18: detection unit; 19; determination unit; 100: pre-filter; 101: filter; 102: filter processed object; 105: biochip.

## Claims

1. An apparatus comprising:
a first filtration unit that filters a fluid sample by a filter;
a culture unit that performs culture using a resultant object that has undergone a processing treatment to reduce a flat size for the filter after filtration by the first filtration unit; and
a detection unit that detects nucleic acids of organisms cultured by the culture unit.

2. The apparatus according to claim 1, further comprising a processing treatment unit that performs the processing treatment on the filter after filtration by the first filtration unit, and produces a filter processed object as the resultant object.

3. The apparatus according to claim 2, wherein the processing treatment unit cuts the filter after filtration by the first filtration unit to produce the filter processed object.

4. The apparatus according to claim 2, wherein the processing treatment unit bends the filter after filtration by the first filtration unit to produce the filter processed object.

5. The apparatus according to claim 2, further comprising:
a second filtration unit that filters a liquid medium after culture using the filter processed object; and
an extraction unit that extracts nucleic acids from a residue after filtration by the second filtration unit,
wherein the detection unit detects nucleic acids extracted by the extraction unit.

6. The apparatus according to claim 5, wherein the extraction unit contains the filter processed object used in filtration by the second filtration unit in a sealed container for heating, and extracts nucleic acids of organisms.

7. The apparatus according to claim 1, further comprising a third filtration unit that filters a fluid sample by a pre-filter with meshes larger than those of the filter before filtration by the first filtration unit.

8. A method comprising:
first filtering for filtering a fluid sample by a filter;
culturing using a resultant object that has undergone a processing treatment to reduce a flat size for the filter after filtration; and
detecting nucleic acids of organisms cultured by the culturing.
